(19) Europäisches Patentamt
European Patent Office
Office européen des brevets

(11) Veröffentlichungsnummer : **0 423 479 B1**

(12) # EUROPÄISCHE PATENTSCHRIFT

(45) Veröffentlichungstag der Patentschrift : **11.08.93 Patentblatt 93/32**

(51) Int. Cl.$^5$ : **C07C 25/18,** C07C 17/12

(21) Anmeldenummer : **90116923.5**

(22) Anmeldetag : **04.09.90**

(54) **Verfahren zur Herstellung von in 4,4'-Stellung dihalogenierten Oligophenylen.**

(30) Priorität : **15.09.89 DE 3930848**

(43) Veröffentlichungstag der Anmeldung : **24.04.91 Patentblatt 91/17**

(45) Bekanntmachung des Hinweises auf die Patenterteilung : **11.08.93 Patentblatt 93/32**

(84) Benannte Vertragsstaaten : **BE DE ES FR GB IT NL**

(56) Entgegenhaltungen :
**EP-A- 0 349 381**
**DE-A- 2 056 441**
**GB-A- 2 155 009**
**US-A- 3 518 316**

(56) Entgegenhaltungen :
**SYNTHESIS Nr. 12, Dezember 1985, Seiten 1157,1158, Stuttgart, DE; K. SMITH: "Highly para-Selective Mono-Chlorination of Aromatic Compounds Under Mild Conditions by t-Butyl Hypochlorite in the Presence of Zeolites"**
**PATENT ABSTRACTS OF JAPAN Band 4, Nr. 101 (C-19)(583), 19. Juli 1980; & JP - A - 5564532 (HODOGAYA KAGAKU) 15.05.1980**

(73) Patentinhaber : **BAYER AG**
**W-5090 Leverkusen 1 Bayerwerk (DE)**

(72) Erfinder : **Botta, Artur, Dr.**
**Bodelschwingstrasse 19**
**W-4150 Krefeld (DE)**
Erfinder : **Buysch, Hans-Josef, Dr.**
**Brandenburger Strasse 28**
**W-4150 Krefeld (DE)**
Erfinder : **Puppe, Lothar, Dr.**
**Am Weiher 10a**
**W-5093 Burscheid (DE)**

EP 0 423 479 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur selektiven Halogenierung von Oligophenylen in der 4,4'-Stellung in Gegenwart von Zeolithen mit Porenweiten von mindestens 5Å und in weiterer Gegenwart von Dichlormethan.

Beidseitig p-ständig halogenierte Oligophenyle, wie 4,4'-Dichloro-, 4,4'-Dibromo-diphenyl oder -terphenyl beanspruchen starkes technisches Interesse, beispielsweise als Zwischenprodukte für hochwärmefeste Kunststoffe, wie Polyphenylensulfid (vgl. JP-Patentanmeldung 61/231 030 bzw. US 3.396.110 [C.A. 69, P 60 564 w]).

Die konventionelle Chlorierung von Diphenyl in Gegenwart von Lewis-Säuren führt zu einer unselektiven statistischen Substitution, wobei das 4,4'-Derivat nicht das bevorzugte ist. So liefert die Chlorierung bei 100° C in Gegenwart von 2,5 Gew.-% $FeCl_3$ eine Selektivität von nur 8 % an dem 4,4'-Dichlorisomeren; der Anteil an Trichlorbiphenyl ist mit 15 % bemerkenswert hoch, wobei bekanntlich polychlorierte Biphenyle zu den hochtoxischen Substanzklassen zählen. Nach den Angaben von US 1.946.040, US 3.226.447 und GB 1.153.746 soll der Zusatz einer Schwefelverbindung bei der Chlorierung von Benzolen die Selektivität zugunsten der para-Substitution erhöhen. Wie das weiter unten aufgeführte Vergleichsbeispiel zeigt, wird aber bei einer solchen Chlorierung von Biphenyl durch Zugabe von 2,5 Gew.-% Thiophen neben 2,5 Gew.-% $FeCl_3$ die Selektivität zugunsten des 4,4'-Dichlorbiphenyls nur unwesentlich gesteigert, während polychlorierte Biphenyle mit mehr als 9 Gew.-% des Reaktionsproduktes immer noch einen beträchtlichen Anteil einnehmen. Diese Tatsache zeigt, daß Biphenyl und Benzol nur sehr begrenzt vergleichbar sind.

Die Chlorierung von p-Terphenyl in Gegenwart von Eisen und Chloroform führt zu einem statistischen Gemisch von o- und p-Monochlor- sowie o,p'- und p,p'-Dichlor-terphenylen, das wegen der Schwerlöslichkeit der p-Isomeren nur mit großem technischem Aufwand zu trennen wäre (Bull. Soc. Chim. France 1968 (10), 4255-58 [C.A. 70 (1969), 57 316 g]).

Die Verwendung von Lewis-Säuren führt in der Praxis weiterhin bekanntlich zu erhöhten Korrosionsproblemen sowie zu einer erschwerten Aufarbeitung und Entsorgung.

Es wurde nun ein Verfahren zur Herstellung von in 4,4'-Stellung dihalogenierten Oligophenylen der Formel

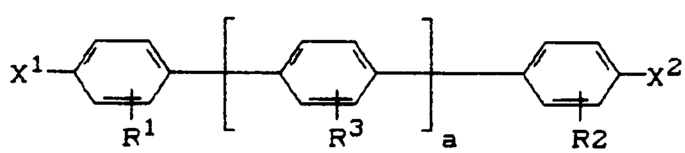 (I)

durch katalysierte Halogenierung von Oligophenylen der Formel

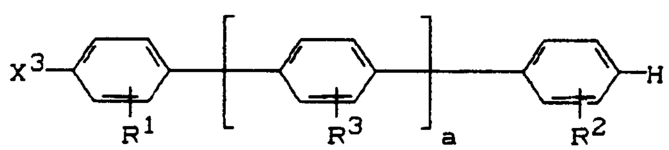 (II),

wobei in den Formeln

$X^1$ und $X^2$ unabhängig voneinander für Chlor oder Brom, bevorzugt für Chlor stehen,

$X^3$ Wasserstoff, Chlor oder Brom, bevorzugt Wasserstoff oder Chlor, besonders bevorzugt Wasserstoff bedeutet,

$R^1$, $R^2$ und $R^3$ unabhängig voneinander für Wasserstoff, $C_1$-$C_4$-Alkyl (bevorzugt Methyl oder Ethyl, besonders bevorzugt Methyl), $C_1$-$C_4$-Alkoxy (bevorzugt Methoxy oder Ethoxy, besonders bevorzugt Methoxy), Fluor, Chlor oder Brom stehen und

a den Wert Null oder Eins annimmt,

mit Halogenierungsmitteln gefunden, das dadurch gekennzeichnet ist, daß in Gegenwart von Dichlormethan oder im wesentlichen Dichlormethan enthaltenden Lösungsmittelgemischen und in Gegenwart von Metallkationen enthaltenden Zeolithen des Strukturtyps L gearbeitet wird.

$C_1$-$C_4$-Alkyl ist beispielsweise Methyl, Ethyl, Propyl, Isopropyl, Butyl, Isobutyl oder tert.-Butyl.

$C_1$-$C_4$-Alkoxy ist beispielsweise Methoxy, Ethoxy, Propoxy, Isopropoxy, Butoxy, Isobutoxy oder tert.-Butoxy.

Für den Fall, daß der Index a den Wert Null annimmt, betrifft die Erfindung die Dihalogenierung von Biphenyl. Für den Fall, daß der Index a den Wert Eins annimmt, betrifft die Erfindung die Dihalogenierung von Terphenylen, in denen die drei Phenylkerne linear verknüpft sind. Die Halogenierung der Oligophenyle substituiert erfindungsgemäß mit außerordentlich hoher Selektivität die zur Ringverknüpfung p-ständige Position.

Die Erfindung betrifft als 4,4'-Dihalogenierung die Chlorierung und die Bromierung, bevorzugt jedoch die Chlorierung. Als Ausgangsmaterialien können die in 4- und 4'-Stellung noch nicht halogenierten Biphenyle oder Terphenyle eingesetzt werden; es ist jedoch auch möglich, im Rahmen der Definition für $X^3$ auch bereits in p-Stellung monohalogenierte Biphenyle oder Terphenyle erfindungsgemäß umzusetzen. Dies hat zum einen Bedeutung für die Recyclisierung von im erfindungsgemäßen Verfahren anfallendem Monohalogenierungsprodukt, zum anderen jedoch für die Tatsache, daß erfindungsgemäß 4,4'-dihalogenierte Biphenyle oder Terphenyle hergestellt werden können, in denen $X^1$ und $X^2$ verschiedenes Halogen darstellen, wenn man ein Monochlor- bzw. Monobrom-oligophenyl einsetzt und zur Einführung des jeweils anderen Halogens erfindungsgemäß mit einem Bromierungsmittel bzw. einem Chlorierungsmittel arbeitet. In bevorzugter Weise ist die Halogenierung der Oligophenyle eine Halogenierung des nicht substituierten Biphenyls bzw. des nicht substituierten und linear verknüpften Terphenyls.

Als Halogenierungsmittel eignen sich Chlor oder Brom sowie Verbindungen, die Chlor oder Brom abgeben, wie Sulfurylchlorid, Sulfurylbromid, N-Chlor-und N-Bromsuccinimid sowie Bromchlor, Bromfluor und andere dem Fachmann bekannte Halogenierungsmittel dieser Art. In bevorzugter Weise wird elementares Chlor bzw. elementares Brom eingesetzt. Besonders bevorzugt ist die Chlorierung mit Hilfe von elementarem Chlor. Das Halogenierungsmittel wird in der Regel im stöchiometrischen Verhältnis zum Oligophenyl eingesetzt, also bei noch nicht in 4- und 4'-Stellung halogenierten Oligophenylen im Molverhältnis 2:1, bei bereits in 4-Stellung monohalogenierten Oligophenylen im Molverhältnis 1:1. Von diesem stöchiometrischen Verhältnis kann bis zu 60 Mol-%, bevorzugt bis zu 40 Mol-%, besonders bevorzugt bis zu 30 Mol-% nach oben oder bis zu 20 Mol-% nach unten abgewichen werden.

Das erfindungsgemäße Verfahren wird in Gegenwart von Zeolithen des Strukturtyps L durchgeführt, bei dem mindestens ein Teil aller austauschbaren Kationen Metallkationen sind. Zeolithe sind kristalline Alumosilikate, die aus einem Netzwerk von $SiO_4$- bzw. $AlO_4$-Tetraedern aufgebaut sind. Die einzelnen Tetraeder sind mit Sauerstoffbrücken über die Ecken untereinander verknüpft und bilden ein räumliches Netzwerk, das von Kanälen und Hohlräumen durchzogen ist. Als Ausgleich für die negative Ladung des Gerüsts sind austauschbare Kationen eingelagert.

Diese Zeolithtypen können mit austauschbaren Kationen aus ihrer Syntheseform oder irgendeiner Vielzahl anderer Kationen im Sinne eines Ionenaustauschs versehen sein. Dieser Austausch ist Stand der Technik und dem Fachmann wohl bekannt. Si und Al in den Zeolithen können zumindest teilweise durch andere Elemente ersetzt sein. Eine ausführliche Darstellung von Zeolithen ist beispielsweise in der Monographie von D. W. Breck "Zeolite Molecular Sieves, Structure, Chemistry, and Use", J. Wiley and Sons, New York, 1974 gegeben.

Der Strukturtyp Zeolith L kann beispielsweise als Kationen solche von Li, Na, K, Rb, Cs, Ca, Mg, Sr, Ba, von Seltenerdmetallen, wie La, Ce, von weiteren Metallen, wie Fe, Zn, Mn, Cr, Co, Ni, Ti, Cu, Ag, Pb oder Gemische von ihnen, bevorzugt Kationen von K, Rb, Cs, Ca, Sr, Ba, Ag, Pb, La oder Gemische von ihnen enthalten. Für den erfindungsgemäßen Einsatz kommt ein Zeolith L in Frage, bei dem 60-100 Äquivalent-%, bevorzugt 80-100 Äquivalent-%, besonders bevorzugt 90-100 Äquivalent-%, aller Kationen Metallkationen sind.

Der Zeolith wird in einer Menge von 1-100 Gew.-%, bevorzugt 3-50 Gew.-%, besonders bevorzugt 5-30 Gew.-%, bezogen auf das Gewicht des umzusetzenden Oligophenyls, eingesetzt.

Die Form des verwendeten Zeolithkatalysators ist für das erfindungsgemäße Verfahren im allgemeinen nicht kritisch. In der Regel - insbesondere bei einer batch-Verfahrensweise - kann er in reiner Form als Pulver eingesetzt werden. Selbstverständlich ist es aber auch möglich - beispielsweise für eine kontinuierliche Verfahrensweise in der Gas-, Flüssig- oder Rieselphase, wo der Katalysator gegebenenfalls in einer stationären bzw. Festbettphase angeordnet wird, ihn in stückiger bzw. geformter Gestalt einzusetzen, um eine bessere Trennung von Reaktionsgut zu gewährleisten. Dabei können übliche, dem Fachmann geläufige Binde- bzw. Formungshilfsmittel mitverwendet werden, die gegenüber dem Halogenierungsagens inert sind, z.B. $SiO_2$, $Al_2O_3$, Tonerde, Graphit, in einer Menge von 0,1 - 80 %, bevorzugt 2-30 %, bezogen auf die Masse des reinen Zeoliths.

Das erfindungsgemäße Verfahren ist insbesondere durch seine Durchführung in Gegenwart von Dichlormethan oder im wesentlichen Dichlormethan enthaltenden Gemischen gekennzeichnet. Es wurde nämlich überraschend gefunden, daß bei der Durchführung in Gegenwart von Dichlormethan Selektivitäten vom in 4,4'-Stellung dihalogenierten Oligophenyl der Formel (I) von mindestens 90 % erreicht werden, die mit anderen Verbindungen, die dem Dichlormethan nahestehen, wie Chloroform oder Tetrachlorkohlenstoff, aber auch mit anderen als Lösungsmittel benutzten Verbindungen nicht erreicht werden. Die hohe Selektivität ist umso überraschender, da der Einsatz von Dichlormethan im erfindungsgemäßen Verfahren bereits in Mengen wirksam

ist, die nicht ausreichen, um das eingesetzte zu halogenierende Oligophenyl vollständig zu lösen und die erst recht nicht ausreichen, um das in der Regel schwerer lösliche Dihalogenierungsprodukt vollständig zu lösen. Demnach beträgt die Menge des eingesetzten Dichlormethans das 0,3-100-fache, vorzugsweise das 0,5-50-fache, besonders bevorzugt das 1-20-fache des Gewichts des eingesetzten Oligophenyls. Die untere Grenze des angegebenen Mengenbereichs ist vor allem durch die verfahrenstechnische Maßnahme gegeben, daß das heterogene Gemisch Zeolith/Ausgangsmaterial/Dichlormethan noch rührfähig ist. Im oberen Teil des angegebenen Mengenbereichs werden auch Zustände erreicht, in denen das Ausgangsmaterial vollständig gelöst ist, aber das dichlorierte Endprodukt nicht mehr völlig gelöst ist und neben dem Zeolith eine zweite feste disperse Phase darstellt.

Neben dem Dichlormethan können andere flüssige Verbindungen, die als Lösungsmittel für Chlorierungsreaktionen bekannt sind, mitverwendet werden Beispiele hierfür sind Kohlenwasserstoffe oder Halogenkohlenwasserstoffe, wie Petrolether, Chloroform, Tetrachlorkohlenstoff, 1,1,1-Trichlorethan, 1,2-Dichlorethan, 1,2-Dibrompropan, Perchlorethan, Perchlorethylen, niedere Carbonsäuren, wie Essigsäure und andere dem Fachmann bekannte Lösungsmittel. Stets ist jedoch das Dichlormethan als der wesentliche Bestandteil eines Gemisches Dichlormethan/anderes Lösungsmittel vorhanden, beispielsweise in einer Menge von 50-100 Gew.-%, bevorzugt 75-100 Gew.-% eines solchen Gemisches. In besonders bevorzugter Weise wird in Dichlormethan allein gearbeitet.

Als Cokatalysatoren für Chlorierungen bekannte Substanzen, beispielsweise aus der Reihe der niederen Alkohole, der niederen Carbonsäuren, der Schwefelverbindungen und/oder der Quartärammoniumsalze können im erfindungsgemäßen Verfahren mitverwendet werden, bringen im allgemeinen aber keine weiteren Vorteile, so daß in bevorzugter Weise auf ihre Mitverwendung verzichtet wird. Für den Fall ihrer Mitverwendung sei eine Menge von 0,02-20 Gew.-%, bevorzugt 0,02-2 Gew.-%, bezogen auf die Menge des Zeolith-Katalysators, genannt.

Die Reaktionstemperatur liegt im allgemeinen im Bereich von 0°C bis +80°C. Der Druck ist für das erfindungsgemäße Verfahren nicht kritisch und dient lediglich zur Einstellung der gewünschten Reaktionstemperatur. So wird beispielsweise im oberen Teil des genannten Temperaturbereichs unter erhöhtem Druck, beispielsweise dem sich einstellenden Eigendruck des Sytems, gearbeitet. Bevorzugt ist die Arbeitsweise unter Normaldruck bei 10-45° C.

Das erfindungsgemäße Verfahren ist demnach dadurch gekennzeichnet, daß durch die Gegenwart des Dichlormethans ein flüssiges Reaktionsmedium vorliegt, in welchem der Zeolith-Katalysator suspendiert ist und in dem das umzusetzende Oligophenyl gelöst oder zum Teil ebenfalls suspendiert vorliegt. Zur Erzielung einer höheren Raum-Zeit-Ausbeute kann von der Tatsache Gebrauch gemacht werden, daß das erfindungsgemäße Verfahren gleichermaßen auch mit einem teilweise suspendierten Ausgangsmaterial erfolgreich durchgeführt werden kann. Eine solche Verfahrensweise, bei der neben dem Zeolith-Katalysator auch ein Teil des Ausgangsproduktes und vor allem das schwerer lösliche Verfahrensprodukt suspendiert vorliegen, ist daher bevorzugt. Gemäß dieser zuletztgenannten Verfahrensvariante wird das erfindungsgemäße Verfahren beispielsweise so durchgeführt, daß das Oligophenyl unter Rühren im Dichlormethan (gegebenenfalls unter Mitverwendung der obengenannten Lösungsmittel) suspendiert wird. Sodann wird der Zeolith-Katalysator in gepulverter oder in granulierter Form zugeführt und danach 2 Mol des Halogenierungsmittels in dem Maße, wie es verbraucht wird, bei Reaktionstemperatur in die flüssig-disperse Phase eingeleitet.

Diese Variante des erfindungsgemäßen Halogenierungsverfahrens gestattet demnach Stoffumwandlungen von einem im Reaktionsmedium weitgehend ungelösten Ausgangsmaterial zu einem ebenfalls weitgehend ungelösten Reaktionsprodukt, wobei bei druckloser Fahrweise und milden Temperaturen die Halogenierung mit großer Geschwindigkeit abläuft. Für die Transportvorgänge in das Poren- und Kanalsystem des ebenfalls im Reaktionsmedium Dichlormethan unlöslichen Zeoliths hinein und wieder heraus sowie für die katalytische formselektive Wirkung des Zeolith-Katalysators ist offenbar die spezielle stoffliche Eigenart des Dichlormethan von ausschlaggebender Bedeutung. Trotz des Vorhandensein mehrerer disperser Phasen treten keine Verstopfungs- oder Desaktivierungserscheinungen am Zeolith-Katalysator auf.

Für eine kontinuierliche Arbeitsweise beim erfindungsgemäßen Verfahren eignen sich beispielsweise Säulenapparaturen, in denen der Zeolith-Katalysator in stückiger bzw. granulierter Form oder in Pulverform auf verschiedenen Böden angeordnet wird. Über eine solche Anordnung werden das Oligophenyl-Dichlormethan-Gemisch und das Halogenierungsmittel entweder im Gleichstrom oder im Gegenstrom geführt. Zweckmäßig wählt man in diesem Falle eine solche Verdünnung an Dichlormethan, daß nicht nur das Edukt Oligobiphenyl, sondern auch das Produkt 4,4'-Dihalogenoligophenyl in Lösung bleiben.

Zur Aufarbeitung des Reaktionsgemisches, in welchem der Zeolith-Katalysator und das 4,4'-Dihalogeno-oligophenyl als disperse feste Phase vorliegen, wird eine Extraktion des Dihalogeno-oligophenyls aus dem Zeolith vorgenommen. Für eine solche Extraktion kann der Zeolith-Katalysator mit einem geeigneten Lösungsmittel insbesondere heiß ausgezogen werden. Lösungsmittel hierfür sind beispielsweise: aliphatische oder aromatische Kohlenwasserstoffe, wie Ligroin, Cyclohexan, Toluol, Xylol, Chlorbenzol, o-Dichlorbenzol; Ester, wie Butylacetat, Methylglykolacetat, oder Amide, wie Dimethylformamid.

Grundsätzlich ist hierfür auch eine zusätzliche, größere Menge Dichlormethan geeignet, welches bei dieser Aufarbeitung ausschließlich in seiner Eigenschaft als Extraktionsmittel eingesetzt wird. Grundsätzlich ist es weiterhin möglich, auch ohne Anwendung höherer Temperaturen durch geeignete Lösungsmittel in geeigneter Menge den Zeolith-Katalysator auszulaugen. Eine noch weitere Variante, die gleichzeitig eine bevorzugte ist, besteht darin, das fertige Reaktionsgemisch in einer geeigneten Druckapparatur durch Anwendung höherer Temperaturen mit dem im Reaktionsgemisch vorhandenen Dichlormethan zu extrahieren. Durch die bei höherer Temperatur, beispielsweise bei 80-100° C, gesteigerte Lösefähigkeit des Dichlormethans für das 4,4'-Dihalogeno-oligophenyl wird eine Trennung vom Zeolith erreicht. Unter Beibehaltung des Druckes und der Temperatur schließt sich sodann eine Druckfiltration an. Aus dem Filtrat scheidet sich nach dem Abkühlen das gewünschte Reaktionsprodukt ab. Es kann vorteilhaft sein, die Reaktionsapparatur mit einer Einrichtung zur Druckfiltration auszurüsten, so daß die erfindungsgemäße Halogenierung, die soeben beschriebene Extraktion und die hierzu gehörige Druckfiltration in einer Apparatur ausgeführt werden können.

Für den Fall einer kontinuierlichen Arbeitsweise, wo das 4,4'-Dihalogenoligophenyl in gelöster Form anfallen kann, wird es nach Einengen, gegebenenfalls unter vermindertem Druck, durch Filtration isoliert.

Da in der Regel das gewünschte 4,4'- bzw- 4,4"-Dihalogeno-oligophenyl eine deutlich geringere Löslichkeit, verglichen mit etwa mitentstehenden halogenierten Oligophenylen anderer Substitutionsmuster, zeigt, hat das durch Auskristallisation aus dem Reaktions- oder dem Extraktionsmedium gewonnene Dihalogeno-oligophenyl eine hohe Reinheit von nahezu 100 %. Zur weiteren Steigerung der Reinheit ist eine Umkristallisation, beispielsweise aus Toluol oder o-Dichlorbenzol, möglich.

Nicht oder unvollständig umgesetzte Ausgangsmaterialien können recyclisiert werden. Der als Extraktionsrückstand verbleibende Zeolith-Katalysator kann im allgemeinen ohne weitere Aktivierung erneut erfindungsgemäß eingesetzt werden. Für den Fall, daß nach mehrmaliger Wiederverwendung eine Aktivitätsminderung beobachtet wird, kann der Zeolith-Katalysator nach einem üblichen Verfahren, beispielsweise durch Calcinierung bei erhöhter Temperatur (400-600° C) reaktiviert werden.

Die Ausbeuten/Selektivitäten für die gewünschten 4,4'-Dihalogeno-oligophenyle liegen bei mindestens 90 %, in der Regel beträchtlich oberhalb von 90 %. Sowohl im Falle des 4,4'-Dichlorbiphenyls als auch im Falle des 4,4"-Dichlor-para-terphenyls wird beispielsweise eine Selektivität von 96-97 % erreicht. Solch hohe Selektivitäten waren aus der Kenntnis des Standes der Technik nicht zu erwarten. Zwar ist bereits die para-Monochlorierung von Benzolen, die mit Chlor, Niederalkyl oder niederem Alkoxy substituiert sind, in Gegenwart von Faujasit bzw. Zeolith L beschrieben worden (EP 112 722, EP 118 851, Stud. Surf. Sci. Catal. (Amsterdam) 28 (1986) 747-754), wobei jedoch nur geringfügig höhere Selektivitäten für die para-Chlorierung, verglichen mit der ortho-Chlorierung, angegeben wurden. Eine zweifache Halogenierung von Mehrkernaromaten in Gegenwart von Zeolithen ist jedoch bisher nicht veröffentlicht worden, weil offenbar ein Vorurteil überwunden werden mußte: zum einen ist die genannte Verschiebung des ortho-para-Verhältnisses nur gering, wenn man Zeolithe statt der konventionellen Chlorierungskatalysatoren ($FeCl_3$/Schwefelverbindungen) anwendet; zum anderen war im Hinblick auf die vergrößerte Anzahl von möglichen Substitutionsmustern bei Mehrkernaromaten mit einer undeutlich ausgeprägten Selektivität und einem schwierig aufzuarbeitenden Vielstoff-Reaktionsgemisch zu rechnen.

Wegen der besonders ausgeprägten Schwerlöslichkeit von in 4,4"-Stellung dihalogenierten Terphenylen der Formel

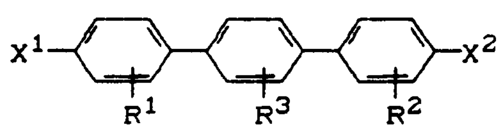

(IV),

in der

$X^1$, $X^2$, $R^1$, $R^2$ und $R^3$ die oben angegebene Bedeutung haben,

ist das erfindungsgemäße Verfahren für deren Herstellung von besonderer Bedeutung, wobei man von Terphenylen der Formel

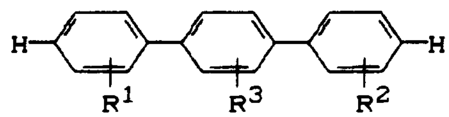

(V)

ausgeht, in der

$R^1$, $R^2$ und $R^3$ die oben angegebene Bedeutung haben.

Hierbei wird in der genannten Weise mit Halogenierungsmitteln in Gegenwart von Metallkationen enthaltenden Zeolithen des Strukturtyps L und weiterhin in Gegenwart von Dichlormethan oder im wesentlichen Dichlormethan enthaltenden Gemischen in den angegebenen Mengen bei den angegebenen Temperaturen gearbeitet, wobei das entstehende Reaktionsgemisch mit dem Zeolith als disperser fester Phase und dem 4,4"-Dihalogeno-terphenyl als weiterer fester Phase zur Gewinnung des 4,4"-Dihalogeno-terphenyl extrahiert wird.

Beispiele

Alle in den folgenden Beispielen genannten Zeolithe wurden vor ihrem Einsatz 2 bis 3 Stunden bei 400°C in einem Muffelofen calciniert.

Beispiel 1

In einem Planschliffreaktor aus Glas (Höhe 28 cm, Durchmesser 11 cm) mit Rührer, Thermometer, Rückflußkühler und einem bis zum Boden reichenden Gaseinleitungsrohr gab man 231,3 g (1,5 Mol) Biphenyl und 1000 ml Dichlormethan zusammen und fügte 45 g pulverförmigen K-Zeolith L zu. Danach wurden unter Rühren bei 40°C insgesamt 234,3 g (3,3 Mol) $Cl_2$-Gas innerhalb von 6 Stunden in die Suspension eingeleitet. Nach etwa der Hälfte der Zeit begann die Ausfällung eines dichten farblosen Niederschlags, der zusätzlich zum Zeolith eine weitere feste disperse Phase darstellte. Nach Einleiten von 90 % (191,7 g), 100 % (213 g) und 110 % (234,3 g) der theoretisch erforderlichen $Cl_2$-Menge wurden Proben zur Bestimmung des Umsatzes entnommen. Wie die folgende Tabelle 1 zeigt (Angaben in Flächen-% der gaschromatographischen Bestimmung), ist der Umsatz jeweils 100 %ig, bezogen auf das eingesetzte Biphenyl. Die Selektivität an 4,4'-Dichlor-biphenyl erreicht bei 110 % der theoretischen Menge an $Cl_2$ einen Wert von etwa 96 %, ohne daß höhere als zweifach-chlorierte Biphenyle gebildet wurden.

Tabelle 1

| Probe bei | Biphenyl | Monochlor-biphenyl | | Dichlor-biphenyl | | |
|---|---|---|---|---|---|---|
| | | 2- | 4- | 2,4'- | 4,4'- | Rest |
| 90 % $Cl_2$ | - | 2,33 | 34,95 | 1,35 | 60,77 | 0,60 |
| 100 % $Cl_2$ | - | 2,26 | 16,09 | 1,83 | 78,92 | 0,88 |
| 110 % $Cl_2$ | - | - | 0,25 | 3,56 | 95,54 | 0,65 |

Beispiele 2 bis 13

In einem 250 ml Dreihalskolben mit Rührer, Thermometer, Gaseinleitungsrohr und Rückflußkühler leitete man innerhalb von 6-9 Stunden bei der angegebenen Reaktionstemperatur in eine Suspension von 38,6 g (0,25 Mol) Biphenyl und 7,5 g Zeolithpulver des angegebenen Typs in 80 ml $CH_2Cl_2$ 35,5 g (0,5 Mol) $Cl_2$ ein. Nach etwa 15 Minuten Nachrührzeit wurde die Zusammensetzung des Reaktionsgemisches gaschromatographisch ermittelt (Tabelle 2). Die Beispiele 2, 3a, 3b und 4 (andere Katalysatoren) sind Vergleichsbeispiele.

<u>Tabelle 2</u>

| Beispiel | Temp. | Zeolith | Biphenyl | Monochlor-biphenyl 2- | 4- | Dichlor-biphenyl 2,4'- | 4,4'- | Rest | Summe 4-/4,4'- |
|---|---|---|---|---|---|---|---|---|---|
| 2 | 20° C | K-Ω | - | 27,52 | 3,38 | 20,85 | 38,01 | 10,24 | 41,39 |
| 3a | 20° C | H-Mordenit | - | 12,41 | 12,28 | 16,18 | 55,27 | 3,86 | 67,55 |
| 3b | 40° C | H-Mordenit | - | 9,57 | 16,14 | 13,78 | 56,15 | 4,36 | 72,29 |
| 4 | 40° C | Rb-Y | - | 32,28 | 20,61 | 12,06 | 25,46 | 9,59 | 46,07 |
| 5 | 20° C | H-L | 0,46 | 23,59 | 2,33 | 25,01 | 38,66 | 9,95 | 40,99 |
| 6 | 40° C | $NH_4$-L | 0,12 | 19,58 | 30,92 | 8,46 | 35,02 | 5,9 | 65,94 |
| 7 | 20° C | Na-L | 0,50 | 2,87 | 42,37 | 1,48 | 52,06 | 0,72 | 94,43 |
| 8 | 20° C | K-L | - | 2,54 | 3,92 | 4,87 | 88,01 | 0,66 | 91,93 |
| 9 | 40° C | Rb-L | 0,05 | 2,15 | 22,72 | 1,65 | 72,37 | 1,06 | 95,09 |
| 10 | 40° C | Ba-L | 0,34 | 4,05 | 17,10 | 2,89 | 74,79 | 0,83 | 91,89 |
| 11 | 40° C | La-L | - | 5,52 | 20,85 | 3,74 | 69,15 | 0,74 | 90,0 |
| 12 | 40° C | Ag-L | 0,12 | 2,71 | 32,29 | 1,72 | 62,70 | 0,46 | 94,99 |
| 13 | 40° C | Pb-L | 0,04 | 2,43 | 8,03 | 2,58 | 86,15 | 0,77 | 94,18 |
| 14 | 40° C | ohne | 38,35 | 11,44 | 35,07 | 0,66 | 1,75 | 14,28 | 36,82 |

Im Reaktionsgemisch vorhandenes 4-Monochlor-biphenyl kann jedoch durch Weiterchlorieren in das 4,4'-Dichlorbiphenyl übergeführt werden. Zur Beurteilung der 4,4'-Selektivität wurde daher in der letzten Spalte von Tabelle 2 die Summe an 4-Mono- und 4,4'-Dichlor-biphenyl aufgeführt.

Beispiel 14

Die Tabelle 2 enthält das Beispiel 14, in welchem unter sonst gleichen Reaktionsbedingungen ohne Zeolith-Katalysator gearbeitet wurde.

Beispiel 15

Das Beispiel 8 wurde wiederholt, wobei jedoch die Menge an Zeolith K-L halbiert wurde (Ergebnisse s. Tab. 3).

Tabelle 3

| Bsp. | Temp. | Zeolith | Kat.-menge | LM | Biphenyl | Monochlor-biphenyl 2- | 4- | Dichlor-biphenyl 2,4'- | 4,4'- | Rest | Summe 4-/4,4'- |
|---|---|---|---|---|---|---|---|---|---|---|---|
| 15 | 20° C | K-L | 10 % | $CH_2Cl_2$ | 0,22 | 3,47 | 9,29 | 3,52 | 82,29 | 1,21 | 91,58 |
| 16 | 20° C | K-L | 20 % | $CHCl_3$ | 0,3 | 19,02 | 30,74 | 8,51 | 32,25 | 9,18 | 62,99 |
| 17 | 60° C | K-L | 20 % | $CHCl_3$ | - | 9,63 | 26,60 | 4,39 | 57,83 | 1,55 | 84,43 |
| 18 | 20° C | K-L | 20 % | $CCl_4$ | - | 12,58 | 7,33 | 13,49 | 60,85 | 5,75 | 68,18 |
| 19 | 40° C | K-L | 20 % | n-Hexan | 9,71 | 11,59 | 54,72 | 2,22 | 18,99 | 2,77 | 73,71 |
| 20 | 20° C | K-L | 20 % | Cyclo-hexan | 0,03 | 8,06 | 5,34 | 13,84 | 67,84 | 4,89 | 73,18 |
| 21 | 20° C | K-L | 20 % | 1,2-Di-chlor-propan | 0,69 | 11,42 | 40,72 | 4,63 | 33,89 | 8,65 | 74,61 |
| 22 | 20° C | K-L | 20 % | 1,1,1-Tri-chlor-ethan | 29,84 | 11,67 | 37,75 | 0,82 | 2,25 | 17,67 | 40,0 |

Beispiele 16 bis 22 (zum Vergleich)

Entsprechend Beispiel 8 wurden Vergleichsbeispiele durchgeführt, bei denen anstelle von $CH_2Cl_2$ andere Lösungsmittel eingesetzt wurden.

Beispiel 23

196 g (2,75 Mol) $Cl_2$ wurden innerhalb von 10 Stunden in eine Aufschlämmung von 288 g (1,25 Mol) p-Terphenyl und 58 g K-Zeolith L-Pulver in 2500 ml Dichlormethan eingeleitet. Dabei erfolgte allmählich eine Umfällung des nicht-zeolithischen Festkörpers. Nach 15-minütigem Nachrühren unter $N_2$-Durchleitung zeigte das Reaktionsgemisch laut gaschromatographischer Analyse die folgende Zusammensetzung:

1,4 % 4-Monochlor-terphenyl
1,9 % 2,4"-Dichlor-terphenyl und
95,7 % 4,4"-Dichlor-terphenyl sowie
1 % unbekannte Komponenten.

Das $CH_2Cl_2$ mit nur geringen Anteilen gelösten Materials aus dem Reaktionsansatz wurde abfiltriert und in dieser Form für einen weiteren Ansatz eingesetzt. Das 4,4"-Dichlor-terphenyl wurde durch Umkristallisation aus o-Dichlorbenzol in einer Reinheit von 99,6-100 % (aus verschiedenen Ansätzen) isoliert. Im Verlaufe der Umkristallisation wurde der Zeolith-katalysator durch Filtration gewonnen, mit Dichlormethan gewaschen und konnte in dieser Form in den nächsten Ansatz ohne Aktivitätsverlust eingesetzt werden.

Beispiele 24 und 25

In einem 500 ml-Dreihalskolben mit Rührer, Gaseinleitungsrohr, Thermometer und Rückflußkühler leitete man in die Suspension von 28,8 g (0,125 Mol) p-Terphenyl und 5,75 g Zeolithpulver des angegebenen Typs in 250 ml $CH_2Cl_2$ innerhalb von 5 Stunden 17,8 g (0,25 Mol) $Cl_2$ bei 40°C ein. Die Ergebnisse der gaschromatographischen Analyse des Reaktionsgemisches sowie die Art des Zeoliths sind in Tabelle 4 aufgelistet.

Beispiele 26 bis 32

Die Versuchsdurchführung erfolgte wie in den Beispielen 24-25, wobei als Katalysator K-L (in Beispiel 31 ohne Katalysator zum Vergleich), als Lösungsmittel $CHCl_3$, $CCl_4$ und $CH_2Cl_2$ bzw. deren Gemische sowie ein $Cl_2$-Überschuß bis ca. 100 % der theoretischen Menge eingesetzt wurden. Proben für die gaschromatographische Analyse wurden je dreimal pro Versuchsbeispiel entnommen, beginnend bei Einleitung eines $Cl_2$-Überschusses von 10 %. Weitere Einzelheiten gehen aus der Tabelle 4 hervor. Die Beispiele 26a-c und 27a-c (kein $CH_2Cl_2$) sind Vergleichsbeispiele.

Tabelle 4 (Angaben in Flächen-% der gaschromatographischen Analyse)

| Beispiel | $Cl_2$-Überschuß | Temp. | Zeolith | LM | Terphenyl | Mono-Cl-Terph. n.b. | Mono-Cl-Terph. n.b. | Mono-Cl-Terph. 4- | Di-Cl-Terph. 2,4"- | Di-Cl-Terph. 4,4"- | Rest | 4-/4,4"-Summe |
|---|---|---|---|---|---|---|---|---|---|---|---|---|
| 24 | 0 % | 40° C | Pb-L | $CH_2Cl_2$ | 2,01 | 0,22 | 0,96 | 56,22 | 2,78 | 37,35 | 0,46 | 93,87 |
| 25 | 0 % | 40° C | Rb-L | $CH_2Cl_2$ | 1,24 | 0,05 | 0,52 | 52,21 | 1,84 | 40,61 | 3,53 | 92,82 |
| 26a | 10 % | 60° C | K-L | $CHCl_3$ | 29,95 | 2,82 | - | 52,77 | 1,13 | 10,33 | 3,0 | 63,1 |
| 26b | 88 % | | | | 11,45 | 3,64 | - | 58,27 | 2,92 | 19,01 | 4,71 | 77,28 |
| 26c | 150 % | | | | 1,69 | 2,80 | - | 52,40 | 5,11 | 32,37 | 5,63 | 84,77 |
| 27a | 10 % | 75° C | K-L | $CCl_4$ | 0,50 | 1,21 | - | 22,98 | 19,98 | 46,37 | 8,96 | 69,35 |
| 27b | 43 % | | | | 0,09 | 0,01 | 8,62 | 4,39 | 22,63 | 50,97 | 13,29 | 55,36 |
| 27c | 69 % | | | | - | - | 4,69 | 4,86 | 22,84 | 49,74 | 17,87 | 54,60 |
| 28a | 10 % | 40° C | K-L | $CH_2Cl_2$/ | 13,29 | 1,27 | - | 63,0 | - | 19,84 | 2,60 | 82,84 |
| 28b | 56 % | | | $CHCl_3$ | 1,72 | 1,52 | - | 58,22 | 3,27 | 33,09 | 2,18 | 91,31 |
| 28c | 117 % | | | 50 : 50 | 0,33 | 1,49 | - | 45,04 | 4,30 | 45,65 | 3,19 | 90,69 |
| 29a | 10 % | 40° C | K-L | $CH_2Cl_2$/ | 0,26 | - | - | 32,18 | 3,63 | 62,82 | 1,11 | 95,00 |
| 29b | 63 % | | | $CHCl_3$ | - | - | - | 4,96 | 4,48 | 89,56 | 1,0 | 94,52 |
| 29c | 101 % | | | 90 : 10 | - | - | - | 1,59 | 4,17 | 93,55 | 0,69 | 95,14 |
| 30a | 10 % | 40° C | K-L | $CH_2Cl_2$/ | - | - | - | 21,94 | 4,48 | 71,70 | 1,88 | 93,64 |
| 30b | 63 % | | | $CHCl_3$ | - | 0,02 | - | 7,46 | 4,11 | 87,25 | 1,16 | 94,71 |
| 30c | 101 % | | | 95 : 5 | - | - | - | 1,24 | 2,87 | 94,80 | 1,09 | 96,04 |
| 31a | 10 % | 40° C | ohne | $CH_2Cl_2$ | 45,63 | 8,73 | - | 32,69 | - | 4,16 | 8,79 | 36,85 |
| 31b | 63 % | | | | 27,51 | 10,38 | - | 43,00 | - | 7,58 | 11,53 | 50,58 |
| 31c | 114 % | | | | 13,72 | 11,28 | - | 48,32 | - | 11,93 | 14,75 | 60,25 |
| 32a | 10 % | 40° C | K-L | $CH_2Cl_2$ | - | - | - | 34,04 | 2,60 | 62,77 | 0,59 | 96,81 |
| 32b | 63 % | | | | - | - | - | 1,65 | 2,48 | 94,68 | 1,19 | 96,33 |
| 32c | 101 % | | | | - | - | - | 1,06 | 1,51 | 95,62 | 1,81 | 96,68 |

Beispiele 33 und 34 (zum Vergleich)

Man brachte 77,1 g (0,5 Mol) Biphenyl in einer Rührapparatur unter Rühren und Durchleiten von Stickstoff zum Schmelzen, fügte 2 g $FeCl_3$ zu und leitete bei 100° C innerhalb von 5 Stunden 71 g (1 Mol) $Cl_2$-Gas ein.

Man hielt noch 30 Minuten bei 100° C, entgaste die Schmelze mit Stickstoff und bestimmte deren Zusammensetzung gaschromatographisch.

In einem weiteren Versuch (Beispiel 34) wurden 2 g $FeCl_3$ und 2 g Thiophen eingesetzt.

Beispiel 35-37

repräsentieren den Einsatz von granuliertem bzw. gebundenem Zeolith L.

In einer Versuchsdurchführung analog Beispiel 1 wurden anstelle von K-Zeolith aus L-Pulver mit Bindemitteln geformte Granulate eingesetzt, wobei der Gehalt an reinem Zeolith L in Beziehung gesetzt wurde:

Beispiel 35: 52,9 g K-L Granulat mit 15 % $SiO_2$

Beispiel 36: 52,9 g K-L Grnaulat mit 15 % $SiO_2$ (analog Beispiel 35, hierbei wurde jedoch das Granulat vorher in einem Mörser gepulvert)

Beispiel 37: 64,3 g K/Na-L Granulat mit 30 % $Al_2O_3$

Die Befunde der gaschromatografischen Analyse sind in Tabelle 6 zusammengefaßt.

Tabelle 5 Produktzusammensetzung

| Beispiel | Monochlorbiphenyl | | Dichlorbiphenyle | | | | | | Trichlorbiph. * | Rest (z.T. unbekannt) |
|---|---|---|---|---|---|---|---|---|---|---|
| | 2- | 4- | 2,2'- | 2,3'- | 2,4'- | 3,4'- | 4,4'- | * | | |
| 33 | 18,9 | 7,89 | 11,7 | 5,73 | 20,97 | 2,24 | 8,04 | 4,66 | 4,63 | 15,3 |
| 34 | 22,8 | 10,51 | 11,1 | 5,48 | 21,34 | 2,41 | 8,61 | 4,87 | 3,19 | 9,7 |

*) unbekannter Substitutionsort

Tabelle 6 Produktzusammensetzung (GG-Flächen-%)

| Beispiel | Probe % $Cl_2$ | Biphenyl | Monochlor-biphenyl 2- | Monochlor-biphenyl 4- | Dichlor-biphenyl 2,4'- | Dichlor-biphenyl 4,4'- | Rest |
|---|---|---|---|---|---|---|---|
| 35 | 90 | - | 2,4 | 22,9 | 1,6 | 73,0 | 0,1 |
| | 100 | - | 2,1 | 4,5 | 2,2 | 91,0 | 0,2 |
| | 110 | - | 0,1 | - | 4,3 | 95,1 | 0,5 |
| 36 | 90 | 0,2 | 3,0 | 36,3 | 1,4 | 58,7 | 0,4 |
| | 100 | - | 3,1 | 19,6 | 2,1 | 74,7 | 0,5 |
| | 110 | - | 1,9 | 1,5 | 3,1 | 73,1 | 0,4 |
| 37 | 90 | 0,1 | 4,3 | 32,5 | 2,7 | 60,1 | 0,3 |
| | 100 | - | 3,9 | 14,0 | 3,6 | 78,1 | 0,4 |
| | 110 | - | 0,9 | 0,4 | 7,2 | 91,0 | 0,5 |

## Patentansprüche

1. Verfahren zur Herstellung von in 4,4'-Stellung dihalogenierten Oligophenylen der Formel

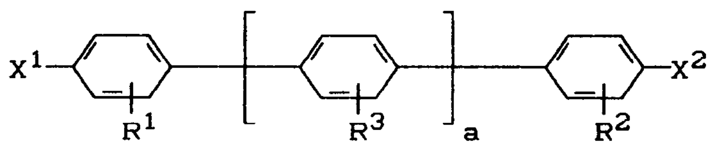

durch Halogenierung von Oligophenylen der Formel

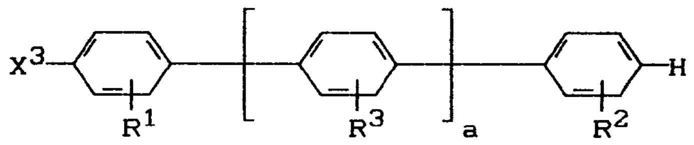

,

wobei in den Formeln

| | |
|---|---|
| $X^1$ und $X^2$ | unabhängig voneinander für Chlor oder Brom, |
| $X^3$ | Wasserstoff, Chlor oder Brom bedeutet, |
| $R^1$, $R^2$ und $R^3$ | unabhängig voneinander für Wasserstoff, $C_1$-$C_4$-Alkyl, $C_1$-$C_4$-Alkoxy, Fluor, Chlor oder Brom stehen und |
| a | den Wert Null oder Eins annimmt, |

mit Halogenierungsmitteln, dadurch gekennzeichnet, daß in Gegenwart von Dichlormethan oder im wesentlichen Dichlormethan enthaltenden Lösungsmittelgemischen und in Gegenwart von Metallkationen enthaltenden Zeolithen des Strukturtyps L gearbeitet wird.

2. Verfahren nach Anspruch 1, dadurch gekennzeichnet, daß die Halogenierung eine Chlorierung ist.

3. Verfahren nach Anspruch 1, dadurch gekennzeichnet, daß 60-100 Äquivalent-% aller Kationen Metallkationen sind.

4. Verfahren nach Anspruch 1, dadurch gekennzeichnet, daß die Metallkationen solche von Li, Na, K, Rb, Cs, Ca, Mg, Sr, Ba, von Seltenerdmetallen, von Fe, Zn, Mn, Cr, Co, Ni, Ti, Cu, Ag, Pb oder Gemische von ihnen sind.

5. Verfahren nach Anspruch 1, dadurch gekennzeichnet, daß bei 0-80°C gearbeitet wird.

6. Verfahren nach Anspruch 1, dadurch gekennzeichnet, daß in einem Reaktionssystem gearbeitet wird, in dem neben dem Dichlormethan als flüssiger Phase und dem Zeolith als disperser fester Phase das Oligophenyl und/oder das 4,4'-Dihalogeno-oligophenyl als weitere disperse feste Phase vorliegen (vorliegt).

7. Verfahren nach Anspruch 6, dadurch gekennzeichnet, daß das Reaktionsgemisch, in welchem der Zeolith-Katalysator und das 4,4'-Dihalogeno-oligophenyl als disperse feste Phase vorliegen, durch Extraktion des 4,4'-Dihalogeno-oligophenyls aufgearbeitet wird.

8. Verfahren nach Anspruch 1, dadurch gekennzeichnet, daß der Index a den Wert Eins annimmt und daß das entstehende Reaktionsgemisch mit dem Zeolith als disperser fester Phase und dem 4,4"-Dihalogeno-terphenyl als weiterer fester Phase zur Gewinnung des 4,4"-Dihalogeno-terphenyls extrahiert wird.

9. Verfahren nach Anspruch 3, dadurch gekennzeichnet, daß 80-100 Äquivalent-% aller Kationen Metallkationen sind.

10. Verfahren nach Anspruch 9, dadurch gekennzeichnet daß 90-100 Äquivalent-% aller Kationen Metallkationen sind.

## Claims

1. Process for the preparation of oligophenyls dihalogenated in the 4,4'-position, of the formula

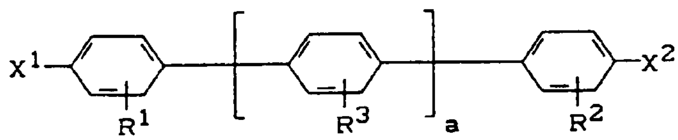

by halogenation of oligophenyls of the formula

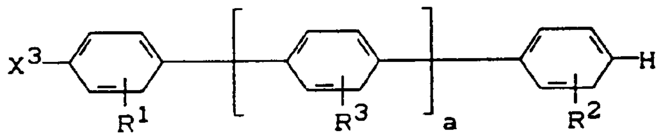

wherein, in the formulae,

| | |
|---|---|
| $X^1$ and $x^2$ | independently of one another represent chlorine or bromine, |
| $x^3$ | denotes hydrogen, chlorine or bromine, |
| $R^1$, $R^2$ and $R^3$ | independently of one another represent hydrogen, $C_1$-$C_4$-alkyl, $C_1$-$C_4$-alkoxy, fluorine, chlorine or bromine and |
| a | assumes the value zero or one, |

with halogenating agents, characterized in that the process is carried out in the presence of methylene chloride or solvent mixtures essentially containing methylene chloride and in the presence of zeolites of the L structure type containing metal cations.

2. Process according to Claim 1, characterized in that the halogenation is a chlorination.

3. Process according to Claim 1, characterized in that 60-100 equivalent % of all the cations are metal cations.

4. Process according to Claim 1, characterized in that the metal cations are those of Li, Na, K, Rb, Cs, Ca, Mg, Sr or Ba, of rare earth metals or of Fe, Zn, Mn, Cr, Co, Ni, Ti, Cu, Ag or Pb, or mixtures of these.

5. Process according to Claim 1, characterized in that it is carried out at 0-80°C.

6. Process according to Claim 1, characterized in that it is carried out in a reaction system in which, in addition to the methylene chloride as the liquid phase and the zeolite as a disperse solid phase, the oligophenyl and/or the 4,4',-dihalogeno-oligophenyl is (are) present as a further disperse solid phase.

7. Process according to Claim 6, characterized in that the reaction mixture in which the zeolite catalyst and the 4,4'-dihalogeno-oligophenyl are present as a disperse solid phase is worked up by extraction of the 4,4'-dihalogeno-oligophenyl.

8. Process according to Claim 1, characterized in that the index a assumes the value one and in that the reaction mixture formed, containing the zeolite as a disperse solid phase and the 4,4"-dihalogenoterphenyl as a further solid phase, is extracted to obtain the 4,4"-dihalogeno-terphenyl.

9. Process according to Claim 3, characterized in that 80-100 equivalent % of all the cations are metal cations.

10. Process according to Claim 9, characterized in that 90-100 equivalent % of all the cations are metal cations.

## Revendications

1. Procédé de préparation d'oligophényles dihalogénés en position 4,4' de formule

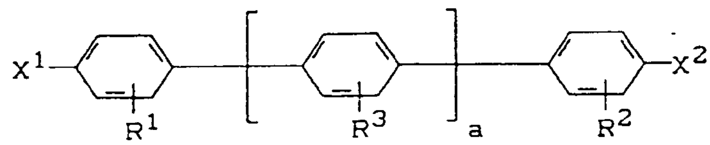

par halogénation d'oligophényles de formule

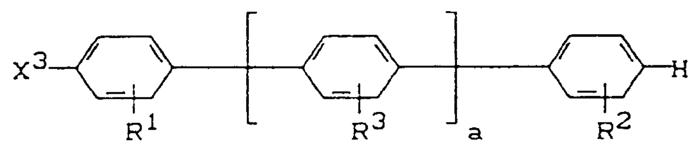

où, dans les formules,
$X^1$ et $X^2$ représentent, indépendamment l'un de l'autre, le chlore ou le brome,
$X^3$ représente l'hydrogène, le chlore ou le brome,
$R^1$, $R^2$ et $R^3$ représentent, indépendamment l'un de l'autre, l'hydrogène, un alkyle en $C_1$-$C_4$, un alcoxy en $C_1$-$C_4$, le fluor, le chlore ou le brome, et
n prend la valeur zéro ou un,
avec des agents d'halogénation, caractérisé en ce que l'on travaille en présence de dichlorométhane ou de mélanges de solvants contenant principalement du dichlorométhane, et en présence de zéolites de type structural L contenant des cations métalliques.

2. Procédé selon la revendication 1, caractérisé en ce que l'halogénation est une chloration.

3. Procédé selon la revendication 1, caractérisé en ce que 60-100 % en équivalents de tous les cations sont des cations métalliques.

4. Procédé selon la revendication 1, caractérisé en ce que les cations métalliques sont ceux de Li, Na, K, Rb, Cs, Ca, Mg, Sr, Ba, de métaux de terres rares, de Fe, Zn, Mn, Cr, Co, Ni, Ti, Cu, Ag, Pb ou des mélanges d'entre eux.

5. Procédé selon la revendication 1, caractérisé en ce que l'on travaille à 0-80°C.

6. Procédé selon la revendication 1, caractérisé en ce que l'on travaille dans un système réactionnel dans lequel, en plus du dichlorométhane comme phase liquide et de la zéolite comme phase solide dispersée, l'oligophényle et/ou le 4,4'-dihalogéno-oligophényle se trouvent sous forme d'une autre phase solide dispersée.

7. Procédé selon la revendication 6, caractérisé en ce que l'on traite le mélange réactionnel, dans lequel le catalyseur zéolite et le 4,4'-dihalogéno-oligophényle se trouvent sous forme d'une phase solide dispersée, par extraction du 4,4'-dihalogéno-oligophényle.

8. Procédé selon la revendication 1, caractérisé en ce que l'indice a prend la valeur un et que l'on extrait le mélange réactionnel formé, contenant la zéolite sous forme de phase solide dispersée et le 4,4"-dihalogénoterphényle sous forme d'une autre phase solide, pour obtenir le 4,4"-dihalogénoterphényle.

9. Procédé selon la revendication 3, caractérisé en ce que 80-100 % en équivalents de tous les cations sont des cations métalliques.

10. Procédé selon la revendication 9, caractérisé en ce que 90-100 % en équivalents de tous les cations sont des cations métalliques.